# EUROPEAN PATENT APPLICATION

(11) **EP 4 109 468 A1**
(43) Date of publication of application: **28.12.2022**
(21) Application number: 21184625.8
(22) Date of filing: 08.07.2021
(51) Int. Cl.: G16H 50/20, G16H 50/30

(54) **SYSTEMS AND METHODS FOR MODELLING A HUMAN SUBJECT**

(30) Priority: 24.06.2021 US 202163214520 P
(71) Applicant: Koninklijke Philips N.V., 5656 AG Eindhoven (NL)
(72) Inventor: Bulut, Murtaza, 5656 AG Eindhoven (NL); Hendriks, Cornelis Petrus, 5656 AG Eindhoven (NL); Cox, Lieke Gertruda Elisabeth, 5656 AG Eindhoven (NL); Lavezzo, Valentina, 5656 AG Eindhoven (NL); Cronie, Harm, 1015 Lausanne (CH)
(74) Representative: Philips Intellectual Property & Standards

(57) **Abstract**

Systems and methods are proposed for verifying a DT of a biological asset (or biological system) of a subject. Such proposals may be based on obtaining: a first signal comprising a value of a physiological parameter of the subject (e.g. determined by a physical and/or virtual sensor arrangement); and a second signal generated by the DT that simulates a value of a physiological parameter of the subject. By comparing the first and second signals, a verification result may then be determined.

## Description

### FIELD OF THE INVENTION

The invention relates to modelling human subjects, and more particularly to the field of models of biological function (commonly referred to as digital twins).

### BACKGROUND OF THE INVENTION

A recent development in healthcare is the so-called 'digital twin' concept. In this concept, a digital representation or computational simulation (i.e. the Digital Twin (DT)) of a physical system is provided and connected to its physical counterpart, for example through the Internet of things as explained in US 2017/286572 A1 for example. Through this connection, the DT typically receives data pertaining to the state of the physical system, such as sensor readings or the like, based on which the digital twin can predict the actual or future status of the physical system, e.g. through simulation.

A DT has been defined as "a set of virtual information constructs that mimics the structure, context and behavior of an individual or unique physical asset that is dynamically
updated with data from its physical twin throughout its life-cycle. Although this definition targets engineering systems, a broad interpretation covers the use of DT in a diverse array of other application areas such as healthcare and information systems. Thus, DTs may be constructed for processes and living entities, and the lifecycle may be the period over which the digital twin is needed to support decision-making. DT may also be used over fixed time periods, such as for surgery or in critical decision points for physical systems. In essence, a digital twin is an in silico model that brings together the technology to map, monitor and control real-world entities by continually receiving and integrating data from a physical twin to provide an up-to-date digital representation of the physical entity.

Such DT technology is also becoming of interest in the medical field, as it provides an approach to more efficient medical care provision. For example, a DT may be built using imaging data of a subject (i.e. patient), e.g. a person suffering from a diagnosed medical condition as captured in the imaging data.

The DT(s) of a subject (i.e. subject-specific computational simulations) may serve a number of purposes. Firstly, the DT(s) (rather than the patient) may be subjected to a number of virtual tests, e.g. treatment plans, to determine which treatment plan is most likely to be successful to the patient. This reduces the number of tests that physically need to be performed on the actual patient. Secondly, the DT(s) of a subject may be used to predict the onset, treatment (outcome) or development of medical conditions of the subject. That is, the DT(s) of a subject may offer a healthcare professionals advanced visualization and/or physical insights into health information of the subject, thus supporting improved Clinical Decision Support (CDS).

DTs are therefore powerful and complex tools with capabilities that can directly influence healthcare options and services that a user can/will receive. Moreover, DTs can have a direct influence on lifestyle choices of a user (e.g. when used outside the healthcare context).

The main components of a DT tool are bio-physical and/or data-driven models that represent the physical object or process. Such models are derived from carefully collected and labelled living organism (e.g. human) data. Such models can be complex in nature and therefore once they are built tracing back their integrity and verifying if they really belong to the living being in question, can be challenging, and typically only possible for the party that has built the DT.

However, a user or owner of a DT may want to make use the DT when receiving care from different healthcare providers. In such circumstances, the healthcare provider will need to verify that the DT does indeed corresponding to that individual before the DT can be used.

### SUMMARY OF THE INVENTION

The invention is defined by the independent claims. The dependent claims define advantageous embodiments.

According to examples in accordance with an aspect of the invention, there is provided a method for verifying a digital twin of a biological system of a subject, the method comprising:
obtaining a sensor signal comprising a value of a physiological parameter of the subject determined by a sensor arrangement
processing input data with the digital twin to generate a simulated signal comprising a simulated value of a physiological parameter of the subject;
comparing the sensor signal and simulated signal; and
determining a verification result based on the comparison result.

Embodiments propose concepts for verifying a DT of a biological system of a subject. Such proposals are based on a realization that signals generated by the DT may be leveraged for verification. That is, proposed are concepts make use of signals generated by the DT.

For example, unlike conventional approaches, embodiment may propose (i) using a DT to generate a test signal to be matched with the templates calculated from the subject's signals, and (ii) using features calculated from the subject's signal to drive and run simulations using the DT, and then DT simulation outputs may be used to generate a match score for example.

Embodiments thus propose one or more concepts for an adaptive verification method which makes use of the DT generated signals. Furthermore, embodiments may also propose using a DT for digital signatures and public key generation. Embodiments may therefore facilitate DT-based biometrics, verification, authentication, identification or digital signatures.

As a result, systems and methods are proposed for DT identity verification. Such proposals may be based on obtaining: a first (i.e. sensor) signal comprising a value of a physiological parameter of the subject (e.g. determined by a physical and/or virtual sensor arrangement); and a second (simulated) signal generated by the DT that simulates a value of a physiological parameter of the subject. By comparing the first and second (i.e. sensor and simulated) signals, a verification result may then be determined.

Embodiments may therefore be based on using biometric information from the subject (e.g. human patient) and from a DT. Such embodiments may cater for the fact that human characteristics can change, thus facilitating adaptation of biometric-based verification.

Proposed embodiments may thus provide dynamic DT verification concepts that cater for changing parameters and/or conditions.

It is also proposed that simulations and/or operations of the DT may be controlled based on a verification result determined according to embodiment. Further, this may be extended to control operations of connected DTs (e.g. based on comparison of their verification results). For example, some embodiments may further comprise modifying the digital twin (or a DT simulation) based on at least one of: the result of comparing the first and second biometric templates; and the verification result.

Embodiments may therefore be of particular use in relation to DTs that support clinical decision making. Exemplary usage applications may for example, securing/authenticating the use of DT for predicting the onset, treatment (outcome) or development of medical conditions and/or medical procedures. Embodiments may thus be of particular use in relation to medical care management and/or prediction.

In some embodiments, comparing the sensor signal and simulated signal may comprise: processing the sensor signal with a feature extraction algorithm to extract a set of sensor signal features from the sensor signal; processing the simulated signal with the feature extraction algorithm to extract a set of simulated signal features from the simulated signal; generating first and second biometric templates based on the set of sensor signal features and the set of simulated signal features, respectively; and comparing the first and second biometric templates.

Determining a verification result may comprise determining a matching score value based on the result of comparing the first and second biometric templates; and determining a verification status of the digital twin based on the matching score value. For example, determining a verification status of the digital twin may comprise: comparing the matching score value against one or more threshold values; and determining whether digital twin is verified based on result of comparing the matching score value against the one or more threshold values.

Some embodiments may further comprise: modifying the input data to generate modified input data; processing the modified input data with the digital twin to generate a second simulated signal comprising a second simulated value of the physiological parameter of the subject; processing the second simulated signal with the feature extraction algorithm to extract a second set of simulated signal features from the second simulated signal; generating a third biometric template based on the second set of simulated signal features; comparing the first and third biometric templates; and determining the verification result further based on the result of comparing the first and third biometric templates. For instance, modifying the input data may comprise modifying the input data based on the determined value of accuracy of the first digital twin.

In some embodiments, the input data may comprise sensor information describing a property of the sensor arrangement.

Some embodiments may further comprise: processing the simulated signal with an authentication method based a second, different digital twin of the biological system of a subject so as to generate an authentication result; and determining the verification result further based on the authentication result.

By way of example, the sensor signal may comprise an historical value of a physiological parameter of the subject previously measured or determined by a sensor arrangement.

According to examples in accordance with yet another aspect of the invention, there is provided a computer program product comprising computer program code means which, when executed on a computing device having a processing system, cause the processing system to perform all of the steps of the method described above.

According to another aspect of the invention, there is provided a system for verifying a digital twin of a biological system of a subject, the system comprising: an input interface configured to obtain a sensor signal comprising a value of a physiological parameter of the subject determined by a sensor arrangement; a processor arrangement configured to process input data with the digital twin to generate a simulated signal comprising a simulated value of a physiological parameter of the subject; a comparison component configured to compare the sensor signal and simulated signal; and a verification component configured to determine a verification result based on the comparison result.

In an embodiment, the comparison component may comprise: a feature extraction component configured to process the sensor signal with a feature extraction algorithm to extract a set of sensor signal features from the sensor signal; and to process the simulated signal with the feature extraction algorithm to extract a set of simulated signal features from the simulated signal; a template generation component configured to generate first and second biometric templates based on the set of sensor signal features and the set of simulated signal features, respectively; and a template comparator configured to compare the first and second biometric templates.

The system may be further configured to generate a control instruction for a sensor or medical equipment based on the determined verification result. In this way, a sensor and/or medical equipment may be controlled according to verification results generated by embodiments. Dynamic and/or automated control concepts may therefore be realized by proposed embodiments.

Further, proposed concepts may provide a clinical decision support comprising a system according to a proposed embodiment.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 is a flow diagram of a method for verifying a digital twin of a biological system of a subject according to a proposed embodiment;
Figure 2 depicts a modification to the method of Figure 1;
Figure 3 is a simplified block diagram of a system according to an embodiment; and
Figure 4 is a simplified block diagram of a computer within which one or more parts of an embodiment may be employed

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides concepts for verifying a DT of a biological asset (or biological system) of a subject. Such concepts may make use of signals generated by the DT itself. Furthermore, concepts are also proposed for using a DT for digital signatures and/or public key generation.

That is, embodiments propose using biometric information from a human subject and from a DT for authentication. Such authentication may be in the context of verifying that a certain DT is the digital model originating from a certain human subject, or part of a body of a certain human subject. Put another way, embodiments may facilitate the verification that a DT (e.g. DT1) models a particular subject (e.g. User1). Such a verification may, for example, be required by a third party (e.g. Hospital 1) before the DT (DT1) is permitted to be used to provide healthcare services to the subject (User1).

Whereas conventional approaches may employ stored templates (i.e. synthesized signals), proposed concepts are based on a realization that the DT may be used to generate a test signal to be matched with a signal from the subject. Proposed concepts may also use features calculated from the subject's signal to drive and run simulations using the DT, and then use DT simulation outputs to generate a match score.

In particular, it is proposed that signals generated by a DT may be used for biometrics. This is because the biometric generation is based on generating (template) signals according to defined processing applied to data collected from the subject(s). This means that even if DT generated signals are not the same as the signals acquired from a human subject, post-processing algorithms for feature extraction and template generation can be adapted such that the results of the processed DT generated signals and human subject signals are comparable. That is, considering the developments in digital modelling of human physiology, it is suggested that the signals generated by DT are very similar to natural human signals. Specifically, when compared one-to-one at sample level there may be still differences, but when compared in terms of features extracted from the signal (as well as shape, temporal or spectral characteristics), it is anticipated that DTs can generate comparable (if not identical) signals to those obtained from a (physical or virtual) sensor arrangement applied to a human subject.

Based on such proposals, embodiments may provide a concept of comparing DT signals with signals from a subject to verify that the DT belongs to the subject. Furthermore, embodiments may also support adapting the DT operation and determining how to use sensor data based on the comparison result(s).

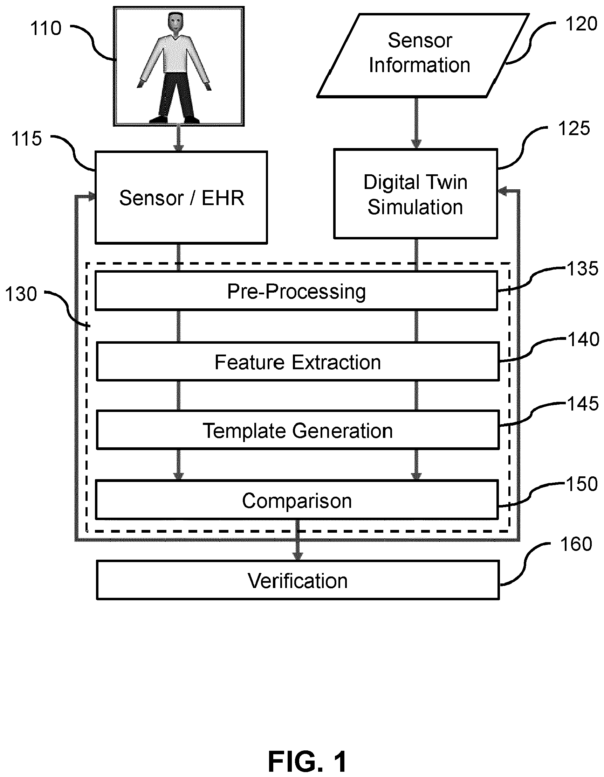

Referring now to Figure 1, there is depicted a flow diagram of a method for verifying a digital twin of a biological system of a subject 110 according to an exemplary embodiment. In this exemplary embodiment, the subject 110 is a human patient and the verification process is configured to verify if the digital twin belongs to the subject (i.e. is configured to model a biological asset of the human patient.

Step 115 comprises obtaining a sensor signal comprising a value of a physiological parameter of the subject determined by a sensor arrangement. Specifically, in this example, the sensor signal comprises a historical value of a physiological parameter of the subject 110 previously determined by a sensor arrangement (e.g. obtained from electronic health records). The sensor arrangement may comprise one or more physical sensors coupled to the subject so as to sense the physical parameter. Alternatively, or additionally, the sensor arrangement may comprise a virtual sensor (i.e. software algorithm) that determines a value of the physiological parameter (e.g. based on obtained and/or detected measurements of the subject).

Step 125 comprises processing input data 120 with the digital twin to generate a simulated signal comprising a simulated value of a physiological parameter of the subject 110. Here, the input data 120 comprises sensor information describing a property of the sensor arrangement (e.g. type of sensor, model of sensor, operating parameters of the sensor, etc.)

Next, the method comprises the step 130 of comparing the sensor signal (from step 115) and the simulated signal (from step 125). Specifically, in the embodiment of Figure 1, the step 130 of comparing the sensor signal and the simulated signal comprises the following four sub-steps 135 through 150:
Step 135: Pre-processing - initial processing of the sensor signal and simulated signal to assist subsequent processing, e.g. noise removal, formatting, filtering, etc.
Step 140: Feature Extraction - processing the sensor signal with a feature extraction algorithm to extract a set of sensor signal features from the sensor signal; and processing the simulated signal with the feature extraction algorithm to extract a set of simulated signal features from the simulated signal;
Step 145 - Template Generation - generating first and second biometric templates based on the set of sensor signal features and the set of simulated signal features, respectively; and
Step 150 - Comparison - comparing the first and second biometric templates.

Finally, in step 160, a verification result is determined based on the comparison result. For example, step 160 of this embodiment comprises: determining a matching score value based on the result of comparing the first and second biometric templates; and determining a verification status of the digital twin based on the matching score value. More specifically, determining a verification status of the digital twin comprises comparing the matching score value against one or more threshold values, and then determining whether digital twin is verified based on result of comparing the matching score value against the one or more threshold values.

It is to be understood that the above-described embodiment of Figure 1 is purely exemplary. Such an embodiment may therefore be adapted and/or modified according to requirements and/or desired application. Also, the method of Figure 1 is only illustrative and the ordering of one or more of the steps may be modified in alternative embodiments. For example, steps 115 and 125 may be undertaken in parallel. Also, some steps may be combined, for example pre-processing, feature extraction and template generation may be undertaken in a single step.

By way of example, it is proposed that simulations and/or operations of the DT may be controlled based on a verification result determined according to an embodiment. Further, this may be extended to control operations of connected DTs (e.g. based on comparison of their verification results). For example, some embodiments may further comprise modifying the digital twin (or a DT simulation) based on at least one of: the result of comparing the first and second biometric templates; and the verification result.

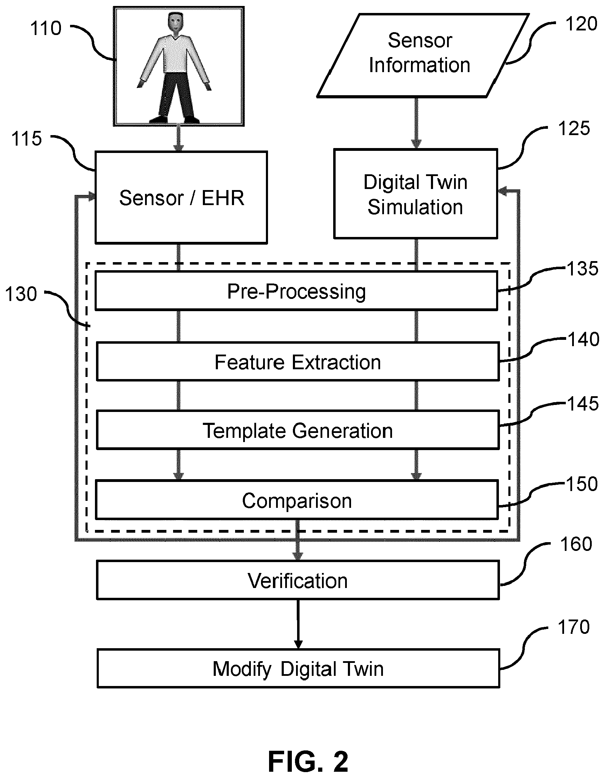

For instance, referring to Figure 2, there is depicted a flow diagram of a method according to another embodiment, wherein the method is a modified version of the method of Figure 1. Specifically, the embodiment of Figure 2 comprises all of the steps of the method of Figure 1, but further comprises the step 170 of modifying the digital twin based on at least one of: the result of comparing the first and second biometric templates; and the verification result.

In a further example, it is proposed that the input data to the DT may be modified in order to generate a further simulated signal which can, in turn, be used as extra information for verification. For instance, an alternative embodiment may further comprise: modifying the input data to generate modified input data; and processing the modified input data with the digital twin to generate a second simulated signal comprising a second simulated value of the physiological parameter of the subject. This second simulated signal can then be processed with the feature extraction algorithm to extract a second set of simulated signal features from the second simulated signal, and a third biometric template then generated based on the second set of simulated signal features. The first and third biometric templates can then be compared, and the verification result then determined further based on the result of comparing the first and third biometric templates. In such an example, the input data may be modified based on the determined value of accuracy of the first digital twin.

According to yet another example, it is proposed that more than one DT may be used. For example, a second reference (or trusted) DT may be employed. For instance, in such an embodiment, the method may comprise the steps of: processing the simulated signal with an authentication method based a second, different digital twin of the biological system of a subject so as to generate an authentication result; and determining the verification result further based on the authentication result.

It is also proposed that location information may be used as additional information during a DT verification process. That is, location information may supplement DT verification. For example, it may be checked (from medical records) that a hay fever attack occurrence overlaps with the location where the subject resides (e.g. at a time there was high hay fever alert at that location).

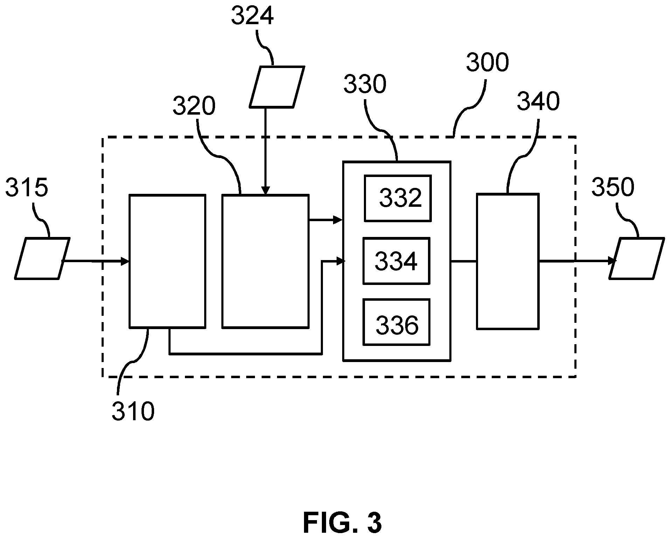

Referring now to Figure 3, there is depicted a simplified block diagram of a system according to an embodiment. The system is for verifying a digital twin of a biological system of a subject according to an exemplary proposal. In this example, the subject 110 is an elderly human requiring regular medical assessment and the verification process is configured to verify if the digital twin belongs to the subject (i.e. is configured to model a biological asset of the human patient).

The system 300 comprises an input interface 310 that is configured to obtain a sensor signal comprising a value of a physiological parameter of the subject determined by a sensor arrangement. Specifically, the sensor signal comprises one or more vital signs detected by one or more sensors coupled to the subject.

A processor arrangement 320 of the system processes input 324 data with the digital twin to generate a simulated signal comprising a simulated value of the physiological parameter of the subject.

The sensor signal and simulated signal are provided to a comparison component 330 of the system 300. The comparison component 330 is configured to compare the sensor signal and simulated signal. Specifically, in this example, the comparison component 330 comprises a feature extraction component 332 configured to process the sensor signal with a feature extraction algorithm to extract a set of sensor signal features from the sensor signal; and to process the simulated signal with the feature extraction algorithm to extract a set of simulated signal features from the simulated signal; a template generation component 334 configured to generate first and second biometric templates based on the set of sensor signal features and the set of simulated signal features, respectively; and a template comparator 336 configured to compare the first and second biometric templates so as to determine a comparison result.

The result of the comparison (i.e. comparison result) is communicated from the comparison component 320 to a verification component 340 of the system 300.

The verification component 340 is configured to determine a verification result 350 based on the comparison result. The verification result 350 is output from the system 300, e.g. for provision to a user and/or external system.

By way of further explanation of the proposed concept(s), examples will now be considered with reference to a person (i.e. subject) X (PX), and organ X (OX) of person X, and a digital twin X (DTX) of the organ X.

There may be the following cases in respect of the relation between OX and DTX:
- Case 1: DTX is almost identical (or acceptable for a given application or usage) replica of OX. Meaning that the DTX output is relevant and matching only to the OX output. That is, DTX is an approximation of OX.
- Case 2: (Possible due to a design choice), the DTX is not an identical copy of OX. For example, it is a digital model built from the data of a group of similar patients. In other words, there is not necessarily one to one match between all signals DTX can generate and the OX. Meaning that the same DTX output can be matching and relevant to more than one person's organ characteristics.
- Case 3: OX has changed (for example due to change in health state of PX), but DTX has not been updated. Meaning that not all the outputs of DTX may be relevant and matching to OX characteristics. Assuming that initially the match between OX and DTX was almost perfect, i.e. as in Case 1, we can argue that in this case outputs of DTX do not match to any other individual (hence the difference from case 2).

Embodiments provide an approach to verifying that DTX belongs to OX, i.e. authentication of the DTX.

### Case 1: DTX =~ OX

The processing steps of the embodiment shown in Figure 1 are undertaken. In particular, on the subject side: Signal 1 is measured using sensor 1; and Signal 1 is processed, features extracted, and then features modified to generate a biometric template. On the DT side, the type of the signal 1 (e.g. ECG) collected by sensor 1 is communicated to DTX; DTX runs simulations to generate the same type of signals - Optionally, at this stage real-time data (different from signal 1) from the subject X can be used as input to the DT. The relevant contextual factors (e.g. activity, medications, etc.) can be also used as input if they are relevant for the generated signal; Multiple DT outputs are generated (e.g. multiple ECG signals); and the DT output(s) processed (features extracted, and biometric template is generated). Here, it is noted that the pre-processing, feature extraction, and template generation blocks are the same for the subject side and the DT side. The obtained biometric templates are then compared (i.e. subject biometric template and the DT biometric template are compared) and a match score is calculated for comparison against one or more thresholds.

The threshold values can be preset based on the known match (i.e. resemblance) between the digital and human twin. For example, if it is known that DTX is designed to be an almost identical replica of OX, then the threshold to verify DTX would be set higher in comparison to another digital twin which is a less good replica of its human twin. The threshold values may also be device or action dependent (e.g. a threshold for general practitioner checkup can be lower than a threshold for surgery).

For example:
If match <= thr_min then DTX verification is unsuccessful.
If match >= thr_max then DTX verification is successful. (Note that, in the most current biometrics implementations thr_min=thr_max)
If thr min < match < thr max then additional DT simulations are run, and new DT signals are generated, and the process is repeated.

After pre-determined number of iterations, if match is still less that thr max then DTX verification is unsuccessful.

In an additional embodiment, instead of calculating a single match score, two or more match scores can be calculated. This means using a comparison algorithm that has been adapted for the corresponding match score. For example, 'False match rate' (i.e. false accept rate): the probability that the system incorrectly matches the input OX pattern to a non-matching DTX template. Also, 'False non-match rate' (i.e. false reject rate): the probability that the system fails to detect genuine match.

In these cases, two categories of input can be generated by DT, respectively:
- Outputs that try to minimize false accept rate: This means that only DT outputs that are known to be of very high accuracy are used.
- Outputs that try to minimize false reject rate: The number of the DT outputs generated and used for matching can be greater, and even when only one of these outputs satisfies the match condition, a match decision can be made.

### Case 2: One-to-many match from DTX to OX

In this case, comparing the DTX templates with the OX templates directly may not be sufficient, because the DTX is only an approximation of the human twin. To verify that the corresponding DT belongs to the user, an alternative comparison and verification approach is employed.

For example, DT models may be recorded and stored with an authorization body. Then, for authorization of DTX, same set of outputs are generated by two digital twins: the authorization body generates one set of outputs using the stored copy, and the DTX (claimed by the user) generates another set of outputs. These outputs are then compared in the same manner as outlined with reference Figure 1, but with the difference that instead of using the user generated signals, signals generated by two DTs (one stored by the authorization body and one stored by the user) are compared.

DT models are recorded and stored with an authorization body. As a result, the authorization body is also able to run and generate DT outputs.

For authorization, DTX and the authorization body generates same type of outputs, for which biometrics are extracted and compared.

It is noted that the comparison and verification approach detailed for this case (Case 2) can also be employed for the verification of Case 1 detailed above.

### Case 3: OX has changed

In this case, because OX has changed, the current DTX is not immediately useful, and the data from OX will not match to the output generated by DTX. However, it may be still desirable to prove that the DTX belongs to OX (for example, researchers can use the DTX to generate a new DT for OX).

To achieve the validation of the DTX, it is proposed to use (past) user electronic health records. Using such electronic health records, the steps described above in relation to Case 1 are followed to verify that the DTX belongs to the individual.

Since here there is no direct involvement of the user (hence, no issue of causing discomfort and annoyance to the user by asking the user to record multiple bio-signals), multiple biological signals from user records can be used for matching computations.

From the above description, it will be appreciated that proposed embodiments facilitate verification of a DT of a biological asset (or biological system) of a subject. However, concepts are also proposed for additional applications/usages. For instance, use of a DT for digital signatures and/or public key generation will be described in the following sections. In such sections, examples will once again be considered with reference to a person (i.e. subject) X (PX), and organ X (OX) of person X, and a digital twin X (DTX) of the organ X.

### - Use of digital twin for digital signatures

The purpose of digital signatures is to verify the sender (who claims to be the owner) of the document. DTX (signals) can be considered as the public key generator, and the user (signals) as private key generator. To create a digital signature, the following steps may be undertaken:
a signal measured from the user is used as a private key; and
the type of the signal is communicated as a part of the digital signature, e.g. as a part of the signed document.

Further, to authenticate the digital signature, the following steps may be undertaken:
DT is used to generate a signal of the same type, which is then used to decrypt, and digest generation; and
The digest is compared with the digest generated from the sent document and the authenticity of the signature is established.

### - Use of digital twin for public key encryption

The purpose here is to verify that a sent document has not changed. In other words, to verify that it is the same document sent by the sender. Here, it is noted that this alone may not verify the authenticity of the sender. For such verification, a digital signature may be needed. For such an example, one may consider the following scenario: A person X that owns DTX wants to get more information from hospital X. The desire may be to ensure that the information sent by the hospital has not been altered.

It is proposed that person X uses his/her biological signals (which can be considered the private key of the human) to generate DTX output. The generated DT output can then be considered as the public key of the individual. This public key is communicated to the hospital, and the hospital then uses the public key to encrypt the clinical information or document that is to be sent to the person X. Once received, person X uses the private key to decrypt hospital's message.

From the above-described embodiments and examples, it will be understood that the proposed concepts may be particularly relevant to medical facilities (e.g. hospitals) where many users may be involved in a subject care process. In particular, embodiments may be of yet further relevance to academic hospitals.

It is to be understood that the above examples and embodiments are only illustrative and the ordering of one or more of the steps may be modified in alternative embodiments.

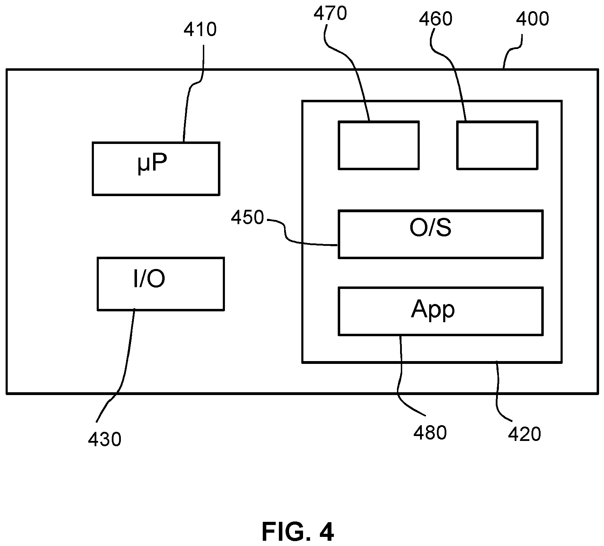

By way of further example, Figure 4 illustrates an example of a computer 400 within which one or more parts of an embodiment may be employed. Various operations discussed above may utilize the capabilities of the computer 400. For example, one or more parts of a system for verifying a DT of a biological asset of a subject may be incorporated in any element, module, application, and/or component discussed herein. In this regard, it is to be understood that system functional blocks can run on a single computer or may be distributed over several computers and locations connected across a cloud-based system (e.g. connected via internet). That is, at least part of the DTs and data may be stored and executed in one or more cloud-based systems, of which the computer 400 may be part.

The computer 400 includes, but is not limited to, PCs, workstations, laptops, PDAs, palm devices, servers, storages, and the like. Generally, in terms of hardware architecture, the computer 400 may include one or more processors 410, memory 420, and one or more I/O devices 430 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 410 is a hardware device for executing software that can be stored in the memory 420. The processor 410 can be virtually any custom made or commercially available processor, a central processing unit (CPU), a digital signal processor (DSP), or an auxiliary processor among several processors associated with the computer 400, and the processor 410 may be a semiconductor based microprocessor (in the form of a microchip) or a microprocessor.

The memory 420 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 420 may incorporate electronic, magnetic, optical, and/or other types of storage media. Note that the memory 420 can have a distributed architecture, where various components are situated remote from one another, but can be accessed by the processor 410.

The software in the memory 420 may include one or more separate programs, each of which comprises an ordered listing of executable instructions for implementing logical functions. The software in the memory 420 includes a suitable operating system (O/S) 450, compiler 460, source code 470, and one or more applications 480 in accordance with exemplary embodiments. As illustrated, the application 480 comprises numerous functional components for implementing the features and operations of the exemplary embodiments. The application 480 of the computer 400 may represent various applications, computational units, logic, functional units, processes, operations, virtual entities, and/or modules in accordance with exemplary embodiments, but the application 480 is not meant to be a limitation.

The operating system 450 controls the execution of other computer programs, and provides scheduling, input-output control, file and data management, memory management, and communication control and related services. It is contemplated by the inventors that the application 480 for implementing exemplary embodiments may be applicable on all commercially available operating systems.

Application 480 may be a source program, executable program (object code), script, or any other entity comprising a set of instructions to be performed. When a source program, then the program is usually translated via a compiler (such as the compiler 460), assembler, interpreter, or the like, which may or may not be included within the memory 420, so as to operate properly in connection with the O/S 450. Furthermore, the application 480 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 430 may include input devices such as, for example but not limited to, a mouse, keyboard, scanner, microphone, camera, etc. Furthermore, the I/O devices 430 may also include output devices, for example but not limited to a printer, display, etc. Finally, the I/O devices 430 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 430 also include components for communicating over various networks, such as the Internet or intranet.

If the computer 400 is a PC, workstation, intelligent device or the like, the software in the memory 420 may further include a basic input output system (BIOS) (omitted for simplicity). The BIOS is a set of essential software routines that initialize and test hardware at startup, start the O/S 450, and support the transfer of data among the hardware devices. The BIOS is stored in some type of read-only-memory, such as ROM, PROM, EPROM, EEPROM or the like, so that the BIOS can be executed when the computer 400 is activated.

When the computer 400 is in operation, the processor 410 is configured to execute software stored within the memory 420, to communicate data to and from the memory 420, and to generally control operations of the computer 470 pursuant to the software. The application 480 and the O/S 450 are read, in whole or in part, by the processor 410, perhaps buffered within the processor 410, and then executed.

When the application 480 is implemented in software it should be noted that the application 480 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 480 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

A single processor or other unit may fulfill the functions of several items recited in the claims.

It will be understood that the disclosed methods are computer-implemented methods. As such, there is also proposed a concept of a computer program comprising code means for implementing any described method when said program is run on a processing system.

The skilled person would be readily capable of developing a processor for carrying out any herein described method. Thus, each step of a flow chart may represent a different action performed by a processor, and may be performed by a respective module of the processing processor.

As discussed above, the system makes use of a processor to perform the data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. Measures recited in mutually different dependent claims can advantageously be combined. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted that the term "adapted to" is intended to be equivalent to the term "configured to". Any reference signs in the claims should not be construed as limiting the scope.

## Claims

1. A method for verifying a digital twin of a biological system of a subject, the method comprising:
obtaining a sensor signal comprising a value of a physiological parameter of the subject determined by a sensor arrangement
processing input data with the digital twin to generate a simulated signal comprising a simulated value of a physiological parameter of the subject;
comparing the sensor signal and simulated signal; and
determining a verification result based on the comparison result.

2. The method of claim 1, wherein comparing the sensor signal and simulated signal comprises:
processing the sensor signal with a feature extraction algorithm to extract a set of sensor signal features from the sensor signal;
processing the simulated signal with the feature extraction algorithm to extract a set of simulated signal features from the simulated signal;
generating first and second biometric templates based on the set of sensor signal features and the set of simulated signal features, respectively; and
comparing the first and second biometric templates.

3. The method of claim 1 or 2, wherein determining a verification result comprises:
determining a matching score value based on the result of comparing the first and second biometric templates; and
determining a verification status of the digital twin based on the matching score value.

4. The method of claim 3, wherein determining a verification status of the digital twin comprises:
comparing the matching score value against one or more threshold values; and
determining whether digital twin is verified based on result of comparing the matching score value against the one or more threshold values.

5. The method of any of claims 1 to 4, further comprising:
modifying the digital twin based on at least one of: the result of comparing the first and second biometric templates; and the verification result.

6. The method of any of claims 1 to 5, further comprising:
modifying the input data to generate modified input data;
processing the modified input data with the digital twin to generate a second simulated signal comprising a second simulated value of the physiological parameter of the subject;
processing the second simulated signal with the feature extraction algorithm to extract a second set of simulated signal features from the second simulated signal;
generating a third biometric template based on the second set of simulated signal features;
comparing the first and third biometric templates; and
determining the verification result further based on the result of comparing the first and third biometric templates.

7. The method of claim 6, wherein modifying the input data comprises:
modifying the input data based on the determined value of accuracy of the first digital twin.

8. The method of any of claims 1 to 6, wherein the input data comprises sensor information describing a property of the sensor arrangement.

9. The method of any of claims 1 to 8, further comprising:
processing the simulated signal with an authentication method based on a second, different digital twin of the biological system of a subject so as to generate an authentication result; and
determining the verification result further based on the authentication result.

10. The method of any of claims 1 to 9, wherein the sensor signal comprises an historical value of a physiological parameter of the subject previously determined by a sensor arrangement.

11. A computer program product comprising computer program code means which, when executed on a computing device having a processing system, cause the processing system to perform all of the steps of the method according to any of claims 1 to 10.

12. A system for verifying a digital twin of a biological system of a subject, the system comprising:
an input interface configured to obtain a sensor signal comprising a value of a physiological parameter of the subject determined by a sensor arrangement;
a processor arrangement configured to process input data with the digital twin to generate a simulated signal comprising a simulated value of a physiological parameter of the subject;
a comparison component configured to compare the sensor signal and simulated signal; and
a verification component configured to determine a verification result based on the comparison result.

13. The system of claim 12, wherein the comparison component comprises:
a feature extraction component configured to process the sensor signal with a feature extraction algorithm to extract a set of sensor signal features from the sensor signal; and to process the simulated signal with the feature extraction algorithm to extract a set of simulated signal features from the simulated signal;
a template generation component configured to generate first and second biometric templates based on the set of sensor signal features and the set of simulated signal features, respectively; and
a template comparator configured to compare the first and second biometric templates.

14. The system of claim 12 or 13, further comprising:
a modification component configured to modify the digital twin based on at least one of: the result of comparing the first and second biometric templates; and the authentication result.

15. A clinical decision support comprising a system for verifying a digital twin of a biological system of a subject according to any of claims 12 to 14.
